# EUROPÄISCHE PATENTSCHRIFT

(11) **EP 1 789 026 B1**
(45) Veröffentlichungstag und Bekanntmachung des Hinweises auf die Patenterteilung: **05.01.2011**
(21) Anmeldenummer: 05766946.7
(22) Anmeldetag: 08.07.2005
(51) Int. Cl.: A61K 9/72, A61P 3/10, A61B 5/08

(54) **VERFAHREN ZUR SYSTEMISCHEN BIOKORREKTUR EINES ORGANISMUS**
METHOD FOR SYSTEMIC BIO-CORRECTION OF AN ORGANISM
PROCEDE DE BIOCORRECTION SYSTEMIQUE D'UN ORGANISME

(30) Priorität: 16.07.2004 DE 102004034640
(43) Veröffentlichungstag der Anmeldung: 30.05.2007
(73) Patentinhaber: Institut für Molekular und, 13125 Berlin (DE)
(72) Erfinder: KUZIN, Alexander, Viktorowitsch, Moskau, 119121 (RU); SCHULZ, Jörg, 13127 Berlin (DE); BENDZKO, Peter, 12623 Berlin (DE); DROGOBYCH, Oleg Wladimirowitsch, Moskauer Gebiet 140070 (RU)
(74) Vertreter: Baumbach, Friedrich
(86) Internationale Anmeldenummer: PCT/DE2005/001224
(87) Internationale Veröffentlichungsnummer: WO 2006/007818

(56) Entgegenhaltungen:
- WO-A-01/00091
- US-B1- 6 387 053

## Beschreibung

Die Erfindung betrifft ein Verfahren zur systemischen Biokorrektur eines Organismus. Anwendungsgebiet der Erfindung ist die Medizin.

### Stand der Technik:

Die Bereitstellung der Energie, die ein Organismus für das Aufrechterhalten seines lebendigen Zustands braucht, beruht auf einer Energiegewinnung infolge einer Vielzahl der chemischen Reaktionen. Die Freisetzung dieser Energie erfolgt durch den Abbau von Kohlenhydraten, Fetten und Eiweißen (Substrate). Die Prozesse der Energiebildung und des Energieverbrauchs laufen in den Mitochondrien ab. Die Energie wird in der Zelle vorwiegend durch die Oxidation dieser Substrate mit Sauerstoff freigesetzt. Ein Teil dieser Energie wird vom Organismus für die Bildung von ATP verwendet.

Die Kopplung der Atmung mit der oxidativen Phosphorylierung erfolgt auf der inneren Mitochondrienmembran. Die Protonen werden über die innere Mitochondrienmembran nach außen gepumpt und so wird ein Protonengradient (pH-Gradient) aufgebaut. Die Protonenbewegung in entgegengesetzter Richtung (durch den Kanal des Faktors Fₒ) führt zur Aktivierung der ATP-Synthetase (Faktor F₁) und der Synthese von ATP aus ADP und Phosphat. Aus der Matrix wird das ATP in das Cytoplasma durch die Translokase transportiert, dieses Enzym katalysiert die Übertragung von einem ATP-Molekül aus der Matrix im Austausch gegen ein Molekül ADP in die Matrix. Die Hemmung des ADP- oder Phosphat-Transports führt zu einer Hemmung der ATP-Synthese.

Es sind folgende Verbindungen bekannt, die die oxidative Phosphorylierung hemmen:
1) Inhibitoren der Dehydrogenasen - ihre Wirkung besteht in der Inhibition der Substratoxidation, wodurch die Bereitstellung des Wasserstoffs in die Atmungskette vermindert wird.
2) Inhibitoren der Atmung - Es entsteht eine Sauerstoffschuld, obwohl Sauerstoff im Überfluss vorhanden ist. Der Protonengradient und die damit verbundene Phosphorylierung werden ausgeschaltet. Es kommt zum energetischen Hunger und die Lebenstätigkeit der Zelle hört auf.
3) Entkoppler der oxidativen Phosphorylierung - sie bewirken eine Protonenübertragung auf dem Umweg über die ATP-Synthetase. Dabei wird die Phosphorylierung eingestellt, so dass nur die Atmung (mit Wärmeproduktion) funktioniert.
4) Inhibitoren der oxidativen Phosphorylierung - hemmen den Protonenfluss über den Fₒ-Kanal und die ATP-Synthese ATP im aktiven Zentrum F₁. Die Phosphorylierung und damit die Atmung hören auf.

Jedes lebende Biosystem deckt seine energetischen Ansprüche über die Außenwelt. Was die energetischen Ansprüche betrifft, so resultieren sie aus den unterschiedlichen, energieabhängigen Prozessen, die für die Arbeit des Biosystems notwendig sind. Deswegen ist es nicht überraschend, dass das lebende Biosystem mit dem ATP einen Botenstoff besitzt, der die Rolle des Vermittlers zwischen der Energiespeicherung und Energiegewinnung spielt. Es wurde lange die Theorie vertreten, dass diese Rolle nur von energiereichen chemischen Verbindungen wie dem Adenosintriphosphat (ATP) übernommen werden kann (Schmidt et al., 2000). Die neuen Erkenntnisse auf dem Gebiet der Bioenergetik zeigen jedoch, dass ein biologisches System über drei Arten von Botenstoffen verfügt. Das sind neben dem wasserlöslichen ATP, das energiegebundene Proton (H⁺) - oder (Na⁺) - Membranpotential. In einem hoch entwickelten Biosystem sind alle drei Arten zu finden. Es existieren Mechanismen, welche die eine Art in eine andere umwandeln können. Für das Überleben braucht das Biosystem jedoch nur eine Reaktionsform, die eine Art produziert (Schmidt et al., 2000).

Für den Ablauf dieser energetischen Prozesse im lebenden Biosystem ist das Verhältnis von Sauerstoffaufnahme und Kohlendioxidabgabe von entscheidender Bedeutung. Der respiratorische Quotient (RQ) ist das Verhältnis der in einer bestimmten Zeitspanne abgegebenen CO₂-Menge zu der in der gleichen Zeiteinheit aufgenommenen O₂-Menge (RQ = VCO₂ / VO₂). Der respiratorische Quotient dient der Berechnung des Energieverbrauchs des Organismus. Bei vollständiger Oxidation von Kohlenhydraten ist VCO₂ = VO_{2,} und RQ = 1. Bei der Fettverbrennung ist relativ mehr O₂ nötig: VCO₂ < VO₂ und RQ = 0.7. Bei einer gleichzeitigen Oxidation von Kohlenhydraten und Fetten liegt der respiratorische Quotient je nach der Zusammensetzung der Nahrung zwischen 1.0 und 0.7. Bei reiner Eiweißverbrennung beträgt der RQ = 0.82, d.h. ist annähernd gleich groß wie bei der Verbrennung einer Mischkost aus Kohlenhydraten und Fetten. Das Verhältnis von Kohlenhydrat- und Fettabbau wird nach dem RQ-Wert aus der Tabelle 1 bestimmt. Weil bei der Oxidation von Kohlenhydraten und Fetten unterschiedliche Energiemengen freigesetzt werden, nimmt das kalorische Äquivalent pro 1 I verbrauchten Sauerstoffes je nach der Kohlenstoffzunahme in der Nahrung (siehe Tabelle 1) zu. Unter Benutzung der RQ-Werte und dem entsprechenden kalorischen Äquivalent pro 1 I O₂ kann der gesamte Energieverbrauch des Organismus aus dem O₂-Verbrauch berechnet werden (indirekte Kalorimetrie).

Um die Fettverbrennung möglichst optimal gestalten zu können, sind neben einer kontinuierlichen Sauerstoffzufuhr und dem Vorhandensein von Antioxidantien, eine Reihe von ungesättigten Fettsäuren wie Omega-3 und Omega-6, essentiell erforderlich (Li, 2003).

**Tabelle 1: RQ-Werte bei unterschiedlicher Kohlenstoff-Fett-Zusammensetzung der Nahrung.**

| RQ | O₂-Verbrauch bei % Oxidation | | Wärmeentwicklung bei % Oxidation | | Kalorisches Äquvalent pro 1 / O₂ | |
|---|---|---|---|---|---|---|
| | KH | Fette | KH | Fette | kJ | Kal |
| 0.70 | 0 | 100 | 0 | 100 | 19.62 | 4.69 |
| 0.75 | 14.7 | 83.3 | 15.6 | 84.4 | 19.84 | 4.73 |
| 0.80 | 31.7 | 68.3 | 33.4 | 66.6 | 20.10 | 4.80 |
| 0.85 | 48.8 | 51.2 | 50.7 | 49.3 | 20.36 | 4.86 |
| 0.90 | 65.9 | 34,1 | 67.5 | 32.5 | 20.61 | 4.92 |
| 0.95 | 82.9 | 17.1 | 84.0 | 16.0 | 20.87 | 4.98 |
| 1.00 | 100 | 0 | 100 | 0 | 21.13 | 5.05 |

Die klinische Relevanz der dargestellten energetischen Vorgänge lässt sich am Metabolischen Syndrom beschreiben. Das Metabolische Syndrom auch als Syndrom X, Insulinresistenz-Syndrom oder Multiples Metabolisches Syndrom bezeichnet, stellt eine Störung der Verstoffwechselung von Kohlenhydraten, Lipiden, Proteinen, Mineralstoffen usw. im Organismus, die durch Erbfaktoren und/oder Lebensbedingungen hervorgerufen werden kann, dar. Zum Metabolischen Syndrom gehören: Insulinresistenz, Hyperinsulinämie, Störung der Glukosetoleranz (Diabetes Typ II), Dyslipidämie, arterielle Hypertonie, koronare Herzkrankheit und andere Herzkreislauferkrankungen, Adipositas, Hyperurikämie (Gicht), Mikroalbuminurie.

Insulinresistenz wird als eine Unempfindlichkeit gegenüber dem körpereigenen Insulin definiert. An der Insulinresistenz leiden nicht nur die Patienten mit Diabetes Typ II, sondern auch ca. 20 % der praktisch gesunden, nicht übergewichtigen Menschen. Die Ursachen der Insulinresistenz sind bis heute noch nicht eindeutig geklärt, zu einem der wichtigsten Risikofaktoren zählt aber der Zustand einer andauernden Hyperglykämie. Zu den Folgen der Insulinresistenz zählen durch die Hyperinsulinämie und Gluko- und Lipotoxität hervorgerufene Erschöpfung von β-Zellen, ihre Desensibilisierung und Apoptose. Dyslipidämie ist eine Störung des Fettstoffwechsels in Form von Hypercholesterinämie, Hypertriglyceridämie, Hyperlipidämie (erhöhte Blutfettwerte als Oberbegriff). Diese ist charakterisiert durch hohe Triglyzerid- und niedrige HDL-Cholesterin-Werte, kleine, dichte LDL-Partikel und einen hohen Gehalt an freien Fettsäuren. Spektrum der Betroffenheit reicht von der "gewöhnlichen" (polygenen) Hypercholesterinämie (ca. 10% der Bevölkerung) bis zu seltenen, genetisch bedingten Lipidstoffwechselstörungen. Dyslipidämien sind Risikofaktoren für die Atherosklerose, insbesondere für die koronare Herzkrankheit. Darüber hinaus tragen die freien Fettsäuren zur Aufrechterhaltung des Zustandes der Insulinresistenz bei.

Die Hyperurikämie ist eine in akuten Schüben oder primär chronisch verlaufende Purin-Stoffwechselstörung, die durch Ausscheidung von harnsauren Salzen an verschiedenen Körperstellen, vorwiegend in den Gelenken (Arthritis urica) und deren Umgebung, charakterisiert ist. Die Veranlagung für Gicht ist zwar angeboren, doch begünstigt wird sie durch Übergewicht, hohen Fleisch- und Alkoholkonsum und Bewegungsmangel. Aus diesen Gründen gilt die Gicht auch als "Wohlstandskrankheit". Man rechnet in eigentlichen "Wohlstandsgebieten" mit ein bis zwei Prozent in der Bevölkerung. Die Folgen der Hyperurikämie sind degenerative Knochenveränderungen und Nephrolithiasis sowie verkürzte Lebenserwartung durch Hypertonie und Nephropathie.

Kardiovaskuläre Erkrankungen und insbesondere die koronare Herzkrankheit (KHK) stellen die prognostisch wesentlichen Komplikationen des metabolischen Syndroms und damit auch die Haupttodesursache dieser Patientengruppe dar.

Die Behandlung des Metabolischen Syndroms im Sinne einer ganzheitlichen Betrachtung ist in der ärztlichen Praxis noch nicht etabliert. Bisher werden einzelne Symptome der Erkrankung (s. o.) als eine selbständige Krankheit behandelt. Die Patienten mit Hyperglykämie und Insulinresistenz werden größtenteils als Diabetiker behandelt, Dyslipidämie-Patienten bekommen cholesterinsenkende Mittel und, im Falle der Fettleibigkeit, eine kalorienarme Diät und Gicht-Patienten sollten auf den Purin-Gehalt der Nahrungsmittel achten.

Es gibt zahlreiche Patente mit der Zielstellung, das Metabolische Syndrom mit Medikamenten zu diagnostizieren und zu behandeln. In EP 835 138 wird vorgeschlagen, Hydrocortisonagonisten wie Dexamethason zur Diagnose einzusetzen. Im kanadischen Patent 2.282.467 werden therapeutischen Präparate beansprucht, die menschliches Wachstumshormon in Kombination mit einem Cortisonagonisten beinhalten. Das russische Patent 2 185 826 schlägt eine Behandlung von Diabetes mellitus Typ 2 mit dem Wirkstoff 2-Ethyl-6-methyl-3-hydroxy-pyridin-succinat vor. Ferner wurde in DE 3687501 T2 vorgeschlagen, die Gamma-Linolensäure und verwandten Verbindungen bei der Behandlung von Komplikationen von Diabetes mellitus zu verwenden. In DE 69909775T2 wird vorgeschlagen, neue Fettsäureanaloga für die Behandlung und/oder Verhinderung von Diabetes zu verwenden.

Die Aufgabe der Erfindung besteht darin, die Energiebilanz der Zelle über eine gezielte Beeinflussung des respiratorischen Quotienten zu optimieren.

### Beschreibung der Erfindung:

Die Erfindung wird gemäß dem Anspruch 1 realisiert, die Unteransprüche sind Vorzugsvarianten.

Der Erfindung liegt die wesentliche Erkenntnis zugrunde, dass das Verhältnis von Sauerstoffaufnahme und Kohlendioxidabgabe in der Atemluft, also der respiratorische Quotient (VCO₂ / VO₂) von 0.72, für die Energiebilanz eines Organismus optimal ist.

Auf dieser Erkenntnis beruht das erfindungsgemäße Verfahren zur systemischen Biokorrektur eines Organismus. Es ist gekennzeichnet dadurch, dass die Energiegewinnung der Zelle durch Einstellung des respiratorischen Quotienten vom anaeroben Kohlenhydratstoffwechsel auf den aeroben Lipidstoffwechsel umgestellt wird. Die Einstellung des respiratorischen Quotienten erfolgt auf einen Wert von 0.70-0.76, welcher als steady-state Bereich bezeichnet wird. Ein bevorzugter Wert für den respiratorischen Quotienten ist 0.71-0.73, besonderes bevorzugt ist ein Quotient von 0.72.

Die wesentliche Maßnahme des oben genannten Verfahrens besteht darin, daß die Einstellung des respiratorischen Quotienten durch Veränderung des Sauerstoffanteils in der eingeatmeten Luft unter physischer Belastung erfolgt. In den meisten zu behandelnden Fällen liegt der respiratorische Quotient oberhalb des Wertes von 0.70-0.76, so dass seine Einstellung durch Zuführung von Sauerstoff in verschiedenen Mengen erfolgt. In selteneren Fällen mit einem Wert < 0.70 wird die Veränderung des Sauerstoffanteils durch Zuführung von Stickstoff geregelt.

Die physische Belastung ist äußerst wichtig, sie erfolgt durch eine länger dauernde und gesteuerte schnelle Bewegung, z. B. auf einem Laufband, einem Fahrrad usw. Die Belastungsintensität wird im Laufe des Verfahrens variiert, wobei die Steuerung durch
- die jeweilige Herzfrequenz
- den jeweiligen respiratorischen Quotienten
- oder durch eine Kombination der jeweiligen Herzfrequenz und des jeweiligen respiratorischen Quotienten variiert wird.

Die Veränderung des Sauerstoffanteils in der Atemluft erfolgt mittels einer Vorrichtung, die die Zufuhr in Abhängigkeit einer optimierten vorgegebenen Herzfrequenz mittels Belastung im steady-state Bereich, bevorzugt bei einer Pulsfrequenz zwischen 120 und 150 min., steuert. Diese Belastung wird für einen Zeitraum von 20-60 Minuten aufrechterhalten und in festgelegten Abständen so oft wiederholt, bis der respiratorische Quotient den Wert von 0,76-0,70 (also den steady-state Bereich) erreicht hat. Gemäß der Erfindung wird der respiratorische Quotient durch eine tägliche Belastung im steady-state Bereich von einer Stunde innerhalb eines Zeitraumes von 2-3 Wochen stabilisiert. Es ist selbstverständlich, dass die physische Belastung für jeden Patienten individuell eingestellt wird, solche individuellen Abweichungen werden jedoch vom Erfindungsanspruch miterfaßt. Die maximale Belastung im steady-state Bereich sollte jedoch eine Herzfrequenz von 120 nicht überschreiten.

Ein wesentlicher Bestandteil der Erfindung liegt darin, dass die Umstellung des Energiegewinns der Zelle durch regelmäßige Zuführung von Omega-3- und Omega-6-Fettsäuren sowie von Antioxidantien und essentiellen Aminosäuren fixiert wird. Vorzugsweise werden 1-2 g Fettsäuren/Tag zugeführt, besonderes bevorzugt 1.5 g/Tag. Ein optimales Verhältnis von Omega-3-Fettsäure zu Omega-6-Fettsäure beträgt 1:1 bis 1:7, wobei ein Verhältnis von 1:5 bevorzugt ist. Als Antioxidantien werden bevorzugt Vitamin E und Vitamin C und als essentielle Aminosäuren bevorzugt Histidin, Isoleucin, Leucin, Lysin, Methionin, Phenylalanin, Threonin, Tryptophan und Valin zugeführt.

Durch die vorliegende Erfindung ist es möglich, eine völlig neuartige Biokorrektur der Zelle zu erreichen, wobei die Energiegewinnung nicht primär aus der Kohlenhydratverbrennung, sondern aus der Fettverbrennung mit dem Effekt der Aufhebung der Insulinresistenz der Zelle erfolgt.

Das erfindungsgemäße Verfahren hat große Bedeutung für durch das Metabolische Syndrom bedingte Erkrankungen. Dazu gehören: Insulinresistenz, Hyperinsulinämie, Störung der Glukosetoleranz (Diabetes Typ II), Dyslipidämie, arterielle Hypertonie, koronaren Herzkrankheit und andere Herzkreislauferkrankungen, Adipositas, Hyperurikämie (Gicht), Mikroalbuminurie.

### Literatur:

Li D.: Omega-3 fatty acids and non-communicable diseases. Chin Med J (Engl). 2003 Mar;116(3):453-8. Review.
Schmidt R.F., Thews G., Lang F.: Physiologie des Menschen (2000), 28. Auflage. Springer-Verlag.

## Patentansprüche

1. Verfahren zur systemischen Biokorrektur eines Organismus, ausgenommen Verfahren zur therapeutischen Behandlung des menschlichen oder tierischen Körpers, **gekennzeichnet dadurch, dass** die Energiegewinnung der Zelle durch Einstellung des respiratorischen Quotienten auf einen Wert von 0.70-0.76, durch Veränderung des Sauerstoffanteils in der eingeatmeten Luft unter physischer Belastung, vom anaeroben Kohlenhydratstoffwechsel auf den aeroben Lipidstoffwechsel umgestellt wird, wobei
- die Veränderung des Sauerstoffanteils in der Atemluft mittels einer Vorrichtung erfolgt, die die Zufuhr in Abhängigkeit einer optimierten vorgegebenen Herzfrequenz mittels Belastung im steady-state Bereich, bevorzugt bei einer Pulsfrequenz zwischen 120 und 150 min., steuert,
- die Belastung im steady-state Bereich für einen Zeitraum von 20-60 Minuten aufrechterhalten und in festgelegten Abständen so oft wiederholt wird, bis der respiratorische Quotient den Wert von 0,76-0,70 erreicht hat, und
- der respiratorische Quotient durch eine tägliche Belastung im steady-state Bereich von einer Stunde innerhalb eines Zeitraumes von 2-3 Wochen stabilisiert wird.

2. Verfahren nach Anspruch 1, **gekennzeichnet dadurch, dass** der respiratorische Quotient auf einem Wert von 0.71-0.73, bevorzugt 0.72 eingestellt wird.

3. Verfahren nach Anspruch 1-2 **gekennzeichnet dadurch, dass** die Veränderung des Sauerstoffanteils bei einem Wert > 0.70-0.76 durch Zuführung von Sauerstoff erfolgt.

4. Verfahren nach Anspruch 1-3, **gekennzeichnet dadurch, dass** die Veränderung des Sauerstoffanteils bei einem Wert < 0.70 durch die Zuführung von Stickstoff erfolgt.

5. Verfahren nach Anspruch 1-4, **gekennzeichnet dadurch, dass** die physische Belastung durch eine länger dauernde und gesteuerte schnelle Bewegung erfolgt, wobei die Belastungsintensität durch den jeweiligen respiratorischen Quotienten gesteuert wird.

6. Verfahren nach Anspruch 1-5, **gekennzeichnet dadurch, dass** die physische Belastung durch eine länger dauernde und gesteuerte schnelle Bewegung erfolgt, wobei die Belastungsintensität durch die jeweilige Herzfrequenz gesteuert wird.

7. Verfahren nach Anspruch 1-6, **gekennzeichnet dadurch, dass** die physische Belastung durch eine länger dauernde und gesteuerte schnelle Bewegung erfolgt, wobei die Belastungsintensität durch die jeweiligen Herzfrequenz und respiratorischen Quotienten variiert wird.

8. Verfahren nach Anspruch 1-7, **gekennzeichnet dadurch, dass** die Belastung mittels eines computergesteuerten Laufbandes erreicht wird.

9. Verfahren nach Anspruch 1-8, **gekennzeichnet dadurch, dass** die physische Belastung individuell eingestellt wird.

10. Verfahren nach Anspruch 1-9, **gekennzeichnet dadurch, dass** die maximale Belastung im steady-state Bereich bei einer Herzfrequenz von 120 liegt.

11. Verfahren nach Anspruch 1-10, **gekennzeichnet dadurch, dass** die Umstellung des Energiegewinns der Zelle durch regelmäßige Zuführung von Omega-3- und Omega-6-Fettsäuren sowie von Antioxidantien und essentiellen Aminosäuren fixiert wird.

12. Verfahren nach Anspruch 11, **gekennzeichnet dadurch, dass** 1-2 g Fettsäuren/Tag zugeführt werden, vorzugsweise 1.5 g/Tag.

13. Verfahren nach Anspruch 11 und 12, **gekennzeichnet dadurch, dass** das Verhältnis von Omega-3-Fettsäure zu Omega-6-Fettsäure 1:1 bis 1:7 beträgt, bevorzugt 1:5.

14. Verfahren nach Anspruch 11, **gekennzeichnet dadurch, dass** als Antioxidantien bevorzugt Vitamin E und Vitamin C zugeführt werden.

15. Verfahren nach Anspruch 11, **gekennzeichnet dadurch, dass** als essentielle Aminosäuren bevorzugt Histidin, Isoleucin, Leucin, Lysin, Methionin, Phenylalanin, Threonin, Tryptophan und Valin zugeführt werden.

## Claims

1. Method for the systemic bio-correction of an organism, with the exception of methods for therapeutic treatment of human or animal bodies, wherein the obtaining of energy by the cell is changed from anaerobic carbohydrate metabolism to aerobic lipid metabolism by setting the respiratory quotient to a value of 0.70-0.76 with a change of the share of oxygen in the inhaled air under physical strain, in which context
- the change of the share of oxygen in the inhaled air is done by means of a device controlling the supply as a function of an optimised, required cardiac frequency by means of strain in the steady-state range, preferably with a pulse frequency between 120 and 150/min.,
- the strain in the steady-state range is maintained for a period of 20-60 minutes and repeated at set intervals until the respiratory quotient has reached the figure of 0.76-0.70, and
- the respiratory quotient is stabilised within a period of 2-3 weeks by a daily strain in the steady-state range of one hour.

2. Method according to Claim 1, wherein the respiratory quotient is set to a value of 0.71-0.73, preferably 0.72.

3. Method according to Claim 1-2, wherein the change of the share of oxygen at a value > 0.70 - 0.76 is done by a supply of oxygen.

4. Method according to Claim 1-3, wherein the change of the share of oxygen at a value < 0.70 is done by a supply of nitrogen.

5. Method according to Claim 1-4, wherein the physical strain is done by a longer-lasting and controlled fast movement, in which context the strain intensity is controlled by the respiratory quotient in question.

6. Method according to Claim 1-5, wherein the physical strain is done by a longer-lasting and controlled fast movement, in which context the strain intensity is controlled by the cardiac frequency in question.

7. Method according to Claim 1- 6, wherein the physical strain is done by a longer-lasting and controlled fast movement, in which context the strain intensity is varied by the cardiac frequency and respiratory quotient in question.

8. Method according to Claim 1-7, wherein the strain is achieved by means of a computer-supported treadmill.

9. Method according to Claim 1-8, wherein the physical strain is set individually.

10. Method according to Claim 1-9, wherein the maximum strain in the steady-state range is at a cardiac frequency of 120.

11. Method according to Claim 1-10, wherein the change of obtaining energy of the cell is fixed by a regular supply of Omega 3 and Omega 6 fatty acids and of antioxidants and essential amino acids.

12. Method according to Claim 11, wherein 1-2 g fatty acids/day are supplied, preferably 1.5 g/day.

13. Method according to Claim 11 und 12, wherein the ratio of Omega 3 fatty acid to Omega 6 fatty acid is 1:1 to 1:7, preferably 1:5.

14. Method according to Claim 11, wherein Vitamin E and Vitamin C are preferably provided as antioxidants.

15. Method according to Claim 11, wherein histidine, isoleucine, leucine, lysine, methionine, phenylalanine, threonine, tryptophan and valine are preferably provided as essential amino acids.

## Revendications

1. Procédé de biocorrection systématique d'un organisme, à l'exception des procédés pour le traitement thérapeutique du corps humain et animal, **caractérisé en ce que** la production d'énergie de la cellule est transférée par ajustement du quotient respiratoire à une valeur de 0.70-0.76 du métabolisme anaérobie des glucides au métabolisme aérobie des lipides par modification de la proportion oxygène dans l'air inspiré sous sollicitation physique, sous respect des conditions suivantes:
- la modification de la proportion oxygène dans l'air respirable est effectuée au moyen d'un dispositif réglant l'alimentation en fonction d'une fréquence cardiaque optimisée spécifiée par sollicitation dans la plage d'état d'équilibre, de préférence à une fréquence du pouls entre 120 et 150 minutes.
- la sollicitation dans la plage d'état d'équilibre est maintenue pour une période de 20-60 minutes et est répétée à des intervalles déterminés autant de fois que nécessaire jusqu'à ce que le quotient respiratoire ait atteint la valeur de 0,76-0,70, et
- le quotient respiratoire est stabilisé par une sollicitation quotidienne dans la plage d'état d'équilibre d'une heure dans une période de 2-3 semaines.

2. Procédé selon la revendication 1, **caractérisé en ce que** le quotient respiratoire est ajusté à une valeur de 0.71-0.73, de préférence à une valeur de 0.72.

3. Procédé selon la revendication 1-2, **caractérisé en ce que** la modification de la proportion oxygène est effectuée à une valeur de > 0.70 - 0.76 par l'alimentation en oxygène.

4. Procédé selon la revendication 1-3, **caractérisé en ce que** la modification de la proportion oxygène est effectuée à une valeur de < 0.70 par l'alimentation en azote.

5. Procédé selon la revendication 1-4, **caractérisé en ce que** la sollicitation physique est effectuée par un mouvement rapide à durée plus longue et réglé, l'intensité de sollicitation étant réglée par le quotient respiratoire respectif.

6. Procédé selon la revendication1-5, **caractérisé en ce que** la sollicitation physique est effectuée par un mouvement rapide à durée plus longue et réglé, l'intensité de sollicitation étant réglée par la fréquence cardiaque respective.

7. Procédé selon la revendication 1- 6, **caractérisé en ce que** la sollicitation physique est effectuée par un mouvement rapide à durée plus longue et réglé, l'intensité de sollicitation étant variée par la fréquence cardiaque respective et le quotient respiratoire.

8. Procédé selon la revendication 1-7, **caractérisé en ce que** la sollicitation est atteinte à l'aide d'un tapis de course commandé par ordinateur.

9. Procédé selon la revendication 1-8, **caractérisé en ce que** la sollicitation physique est ajustée individuellement.

10. Procédé selon la revendication 1-9, **caractérisé en ce que** la sollicitation maximale dans la plage d'état d'équilibre s'effectue à une fréquence cardiaque de 120.

11. Procédé selon la revendication 1-10, **caractérisé en ce que** le transfert de la production d'énergie de la cellule est fixé par l'alimentation régulière d'acides gras oméga 3 et oméga 6 ainsi que d'antioxydants et d'acides aminés essentiels.

12. Procédé selon la revendication 11, **caractérisé en ce que** 1,2 g d'acides gras/jour sont alimentés, de préférence 1,5 g/jour.

13. Procédé selon la revendication 11 et 12, **caractérisé en ce que** la proportion d'acide gras oméga 3 par rapport à l'acide gras oméga 6 est de 1:1 jusqu'à 1:7, de préférence de 1:5.

14. Procédé selon la revendication 11, **caractérisé en ce que** la vitamine E et la vitamine C sont alimentées de préférence comme antioxydants.

15. Procédé selon la revendication 11, **caractérisé en ce que** l'histidine, l'isoleucine, la leucine, la lysine, la méthionine, la phénylalanine, la thréonine, le tryptophane et la valine sont alimentées comme acides aminés essentiels.
